# EUROPÄISCHE PATENTSCHRIFT

(11) **EP 2 565 633 B1**
(45) Veröffentlichungstag und Bekanntmachung des Hinweises auf die Patenterteilung: **13.06.2018**
(21) Anmeldenummer: 11179996.1
(22) Anmeldetag: 05.09.2011
(51) Int. Cl.: G01N 25/04, G01B 11/16, G01N 33/42

(54) **Verfahren zur Bestimmung des Erweichungs- oder Tropfpunkts**
Method for determining softening or dropping point
Procédé de détermination du point de ramollissement ou de goutte

(43) Veröffentlichungstag der Anmeldung: 06.03.2013
(73) Patentinhaber: Mettler-Toledo GmbH, 8606 Greifensee (CH)
(72) Erfinder: Crevatin, Mario, 8409 Winterthur (CH); Tuor, Markus, 8172 Niederglatt (CH); Arnold, Benjamin, 8404 Winterthur (CH); Wildbolz, Pascal, 7402 Bonaduz (CH); Scherrer, Daniel, 8052 Zürich (CH)
(74) Vertreter: Mettler-Toledo

(56) Entgegenhaltungen:
- GB-A- 2 464 717
- JP-A- 58 062 551
- US-A- 426 160
- US-A- 3 242 277
- US-A- 3 587 293
- US-A1- 2009 190 626
- US-B1- 6 536 944
- ROEMER D ET AL: "An Apparatus for the Automatic Determination of Ubbelohde's Flow-Drop-Point of Waxes", FETTE Â SEIFEN Â ANSTRICHMITTEL,, vol. 69, no. 9, 1 January 1967 (1967-01-01), pages 646-651, XP001321285,

## Beschreibung

Die Erfindung betrifft ein Verfahren zur Bestimmung des Erweichungs- oder Tropfpunkts einer Substanz und insbesondere ein automatisiertes Verfahren.

Die Bestimmung des Erweichungs- oder Tropfpunkts ist ein allgemein anerkanntes Verfahren zur Bestimmung von charakteristischen Eigenschaften eines Stoffes oder einer Substanz. US 3,587,293 A offenbart, dass die Bestimmung des Erweichungspunkts insbesondere für Substanzen relevant ist, welchen keinen scharfen Phasenübergang zwischen fest und flüssig aufweisen.

Der Tropfpunkt bezeichnet die Temperatur, bei der eine Substanz unter vorgegebenen und häufig genormten Prüfbedingungen zu fließen beginnt. Dabei wird die Substanz so lange kontrolliert erwärmt, bis diese vom festen in den flüssigen Aggregatzustand übergeht. Während der Messung wird die Temperatur erfasst, bei der sich ein erster Tropfen von der Substanz löst. Die Tropfpunktbestimmung kann gemäss einer der gängigen Normen, wie beispielsweise ASTM D3954, Ph. Eur. 2.2.17 (Pharmacopoea Europaea) oder AOCS Cc 18-18 (American Oil Chemists' Society), durchgeführt werden. Insbesondere bei Polymeren, Rohpolymer, Wachsen, Polyolefinen, Paraffinen, Schmierfetten, organischen Pulvern, Vaseline, Speisefetten, Speiseölen und verwandten Stoffen wird diese zur Charakterisierung sowie auch zur Qualitätskontrolle eingesetzt.

Der Erweichungspunkt ist ein Messwert zur Klassifizierung von beispielsweise Bitumen und bituminösen Substanzen. Der Erweichungspunkt kann mittels eines als Ring-und-Kugel bezeichneten Verfahrens bestimmt werden, wie es beispielsweise in US 3,242,277 A beschrieben ist. Bei der Ermittlung dieses Wertes wird eine Stahlkugel auf eine in einen Ring eingebrachte Bitumenschicht oder Bitumenprobe gelegt. Im Laufe des Versuches wird das Material gleichmäßig erwärmt und die entsprechende Temperatur als Erweichungspunkt festgehalten, bei der sich die Probe um 25,4±0,2 Millimeter nach unten durchgebogen hat. Weiterhin kann der Erweichungspunkt bestimmt werden, indem die Temperatur bestimmt wird, bei welcher ein sich ausdehnender Tropfen eine bestimmte Länge erreicht hat. Die Tropfenbildung aus der zu analysierenden Substanz wird durch Erwärmung hervorgerufen. Der Erweichungspunkt kann gemäss einer der gängigen Normen, wie beispielsweise ASTM D3104, D3461 und D6090 sowie DIN 51920, bestimmt werden. Die Bestimmung des Erweichungspunkts wird ebenfalls zur Qualitätskontrolle sowie zur Charakterisierung von Bitumen, Pech, Asphalt, Harzen, Bindemitteln und verwandten Substanzen eingesetzt.

GB 2464717 A offenbart beispielsweise eine Vorrichtung zur optische Erfassung des Schmelzpunkts, bei der eine Probe in einem Schmelzpunktröhrchen beim Erwärmen visuell erfasst wird und diese Daten zur Ermittlung des Schmelzpunkts ausgewertet werden. Der Schmelzpunkt bezeichnet die Temperatur der Phasenübergangs fest zu flüssig und kann visuell gut verfolgt werden, da eine fest Probe in der Regel lichtundurchlässig und eine flüssige Probe häufig lichtdurchlässig ist, so dass das Erreichen eines Intensitätsmaximum mit dem Erreichen des Schmelzpunkts im Wesentlichen gleichgesetzt werden kann.

Auch US 2009/0190626 A1 offenbart eine Vorrichtung zur optischen Erfassung des Schmelzpunkts und weiterer Phasenübergänge einer oder mehrere Substanzen, welche in den Vertiefungen eines flachen Trägers angeordnet sind. Mit dieser Vorrichtung können vor allem vergleichende Werte für verschiedene Substanzen und weniger absolute Werte für die Phasenübergänge erfasst werden.

Die zu analysierende Probe oder Substanz kann flüssig oder fest sein sowie einen Zwischenzustand umfassen. Beispielhaft kann die Substanz als flüssig, fest, wachsartig, bituminös, zähflüssig oder pulverförmig beschrieben werden.

Bislang sind vor allem Messgeräte bekannt mit denen entweder der Erweichungspunkt oder der Tropfpunkt bestimmt werden kann. Die Bestimmungen erfolgen häufig in Messgeräten mit einer beheizbaren Probenkammer. Die zu analysierende Substanz wird in ein geeignetes Probengefäss gefüllt und dieses wird in die Probenkammer eingebracht, in welcher die Probe, insbesondere die Substanz im Probengefäss, anschliessend erwärmt wird.

Das Erreichen des Erweichungspunktes wird ermittelt, wenn der erste Tropfen der Substanz eine vorgegebene Länge erreicht und zum Beispiel eine entsprechend angeordnete Lichtschranke durchbricht. Die Temperatur zu diesem Zeitpunkt wird als Erweichungspunkt registriert.

JP 58062551 A offenbart ein Gerät für die optische Bestimmung des Erweichungspunktes, bei welcher der Schattenwurf des Tropfens erfasst und dessen Länge ausgewertet wird.

Das Erreichen des Tropfpunktes wird ermittelt, wenn der ersten Tropfen von der Probe abfällt. Dieses Ereignis kann ebenfalls mittels einer Lichtschranke detektiert und die entsprechende Temperatur als Tropfpunkt erfasst werden.

Die Messkammern bekannter Messgeräte sind meist geschlossen und können nicht direkt eingesehen werden. Dieses dient vor allem der Vermeidung von Kältebrücken an einem Fenster und somit der gleichmässigen Erwärmung der Probe. In weiteren bekannten Messgeräten werden Öl- oder Wasserbäder zum Erwärmen der Probe eingesetzt.

Roemer et al., "Eine Apparatur zur automatischen Bestimmung des Fließ-/Tropfpunktes von Wachsen nach Ubbelohde" (FETTE-SEIFEN-ANSTRICHMITTEL, Bd. 69, Nr. 9, 1. Januar 1967, Seiten 646-651) offenbart die Bestimmung des Erweichungs- und Tropfpunkts einer Substanz, mit folgenden Schritten:
- Bereitstellen eines Probengefässes mit einer Probe der Substanz, und Einbringen des Probengefässes in einer Probekammer;
- Einstellen der Probenkammer auf eine Strattemperatur;
- Aufheizen der Probenkammer mittels einer Heizvorrichtung gemäß vorgegebenen Temperatur-Zeit-Sollwerten;
- Messen von Temperatur-Zeit-Istwerten in der Probenkammer mit einem Temperaturfühler und erfassen von Daten aus dem Inneren der Probenkammer mit optischen Mitteln;
- Ermitteln der zeitlichen Veränderungen der Probe in Abhängigkeit der Temperatur anhand der erfassten Daten aus dem Inneren der Probenkammer und Temperatur-Zeit-Istwerte, welche die Bildung mindestens eines Tropfens sowie dessen Längenänderung und Abreißen aufgrund einer Erweichung der Probe durch die Wärmezufuhr der Heizvorrichtung umfasst; und
- Ermitteln des Tropfpunkts und des Erweichungspunkts der Probe anhand ihrer zeitlichen Veränderung in Abhängigkeit der Temperatur.
Die normkonforme Bestimmung des Erweichungspunkts ist jedoch nur für Substanzen durchführbar, welche sich auch normkonform verhalten, also solche welche beim Erwärmen einen Tropfen der gewünschten Länge ausbilden. Für Substanzen, die beispielsweise beim Erwärmen zwar einen Tropfen ausbilden, welcher jedoch zu früh, also vor Erreichen der vorgegebenen Länge, abreisst oder welcher Tropfen die vorgegebene Länge nicht erreicht, sondern zuvor verhärtet, kann kein Erweichungspunkt bestimmt werden.

Daraus ergibt sich als Aufgabe die Bereitstellung eines Verfahrens sowie eines Messgeräts welches die Reproduzierbarkeit der Bestimmung des Tropf- und/oder Erweichungspunkts normkonformer Substanzen verbessert und welches zudem die Analyse von nicht-normkonformen Substanzen ermöglicht.

Gelöst wird diese Aufgabe durch ein, im Anspruch 1 definiertes Verfahren zur Bestimmung des Erweichungs- oder Tropfpunkts einer Substanz mit einem Messgerät. Das Messgerät umfasst eine Probenkammer, einen Temperaturfühler, eine Heizvorrichtung, ein Mittel zur Bereitstellung von Temperatur-Zeit-Sollwerten, ein Mittel zur Bilderfassung, insbesondere einem Mittel zur digitalen Bilderfassung, und eine Steuereinheit mit einer Prozessoreinheit. Die Heizvorrichtung dient zum Erwärmen der Probenkammer anhand vorgegebener Temperaturen, wie den Temperatur-Zeit-Sollwerten. Mit dem Temperaturfühler kann die Temperatur im Inneren der Probenkammer erfasst werden und mit dem Mittel zur Bilderfassung kann das Innere der Probenkammer visuell erfasst werden.

Das erfindungsgemässe Verfahren umfasst mehrere Schritte. Zunächst wird ein Probengefäss mit einer Probe der zu analysierenden Substanz bereitgestellt und in die Probenkammer eingebracht. Weiterhin werden Temperatur-Zeit-Sollwerte bereitgestellt. In der Probenkammer wird eine vorgegebene Starttemperatur eingestellt und anschliessend die Probenkammer gemäss den vorgegebenen Temperatur-Zeit-Sollwerten aufgeheizt. Während der Messung werden Temperatur-Zeit-Istwerte in der Probenkammer mit dem Temperaturfühler ermittelt und zudem Bild-Zeit-Daten aus dem Inneren der Probenkammer mit dem Mittel zur Bilderfassung erfasst. Anhand der erfassten Bild-Zeit-Daten und Temperatur-Zeit-Istwerte kann eine zeitliche Veränderung der Probe in Abhängigkeit der Temperatur ermittelt werden. Die zeitliche Veränderung der Probe umfasst eine Bildung mindestens eines Tropfens sowie dessen Längenänderung oder Abreissen aufgrund einer Erweichung der Probe durch die Wärmezufuhr der Heizvorrichtung (11), welche in den Bild-Zeit-Daten wiedergegeben wird. Anhand der zeitlichen Veränderung der Probe in Abhängigkeit der Temperatur kann deren Tropf- oder Erweichungspunkt ermittelt werden.

Die Auswertung der erfassten Temperatur-Istwerte und Bild-Zeit-Daten ermöglicht die Erfassung des Tropf- oder Erweichungspunktes der Substanz. Zudem kann über den Verlauf der Temperatur-Zeit-Istwerte sowie der aus den Bild-Zeit-Daten ermittelten Änderung der Probe zudem auf weitere rheologische Werte, wie beispielsweise die Viskosität zurückgeschlossen werden.

Die Bilderfassung ermöglicht zudem eine optische Kontrolle der Substanz während des Erwärmungsprozesses, wodurch die Reproduzierbarkeit der Messergebnisse erhöht werden kann, da Messfehler aufgrund von sich atypisch verhaltenden Proben leicht erkannt werden können. Solche Messfehler können beispielsweise auftreten, wenn bei der Bestimmung des Erweichungspunkts der entstehende Tropfen vor Erreichung der vorgegebenen Länge abreisst oder diese gar nicht erst erreicht, oder wenn bei der Tropfpunkt-Bestimmung Tropfen sehr unterschiedlicher Grösse, Form oder Farbe entstehen, was darauf hindeuten kann, dass die Probe sich ungleichmässig erwärmt, bzw. sich die Wärme ungleichmässig in der Probe ausbreitet. Die Reproduzierbarkeit der Messungen kann erhöht werden, indem das unterschiedliche Verhalten von mindestens zwei identischen Proben optisch und/oder visuell verfolgt werden kann und so Abweichungen zwischen den eigentlich identischen Proben erkannt werden können.

Insbesondere für die Bestimmung des Erweichungspunkts besteht ein weiterer Vorteil darin, nicht-normkonformer Substanzen exakter messen zu können. Zu diesen zählen Substanzen deren Eigenspannung beim Erweichen abnimmt, so dass ein Tropfen von der Probe abreisst, bevor er die vorgegeben Länge erreicht hat, oder Substanzen deren Eigenspannung so hoch ist, dass sich kein Tropfen der vorgegebenen Länge ausbildet, sondern dieser vielmehr nur eine verkürzte Länge erreicht.

Das erfindungsgemässe Verfahren umfasst zudem einen Schritt eines Farb- und/oder Weissabgleichs welcher dazu dient das Farbspektrum des Mittels zur Bilderfassung bzw. des Bilderfassungsmittels anzupassen und/oder einzustellen. Dazu kann in der Probenkammer ein Bereich mit einer definierten Farbe, vorzugsweise weiss, angeordnet sein. Dieser Bereich wird vor dem Einbringen der Proben erfasst und das Mittel zur Bilderfassung derart eingestellt, dass das erfasste Bild dieses Bereiches einer vorgegebenen Farbe entspricht, beispielsweise R 100 G 100 B 100 des RGB Farbschemas oder einer anderen definierten Farbe. Der Farb- und/oder Weissabgleich ermöglicht die Erfassung von vergleichbaren Bild-Zeit-Daten, welche insbesondere aufgrund der gezeigten Farben vergleichbar sind. Auf diese Weise können beispielsweise bei Reihenmessungen Farbunterschiede zwischen den Proben erkannt werden, was für den Benutzer vorteilhaft sein kann, da unterschiedliche Farben auf eine unterschiedliche Zusammensetzung der zu analysierenden Substanz oder auch auf eine Zersetzung der Substanz hinweisen können.

Vorzugsweise umfasst das erfindungsgemässe Verfahren zudem einen Helligkeitsabgleich, wodurch sichergestellt werden soll, dass die Bild-Zeit-Daten bei im Wesentlichen gleichen Helligkeitsverhältnissen erfasst werden. Auf diese Weise kann die Reproduzierbarkeit von Messungen gleichartiger Proben zu verschiedenen Zeitpunkten verbessert werden. Der Helligkeitsabgleich kann auf unterschiedliche Weise erfolgen.

Eine Möglichkeit besteht darin die Helligkeit einer regelbaren Lichtquelle anzupassen, welche die Probenkammer beleuchtet. Die Helligkeit wird derart angepasst, dass das Mittel zur Bilderfassung im Wesentlichen gleichartig ausgeleuchtete Bild-Zeit-Daten erfasst.

Eine weitere Möglichkeit besteht darin die Belichtungszeiten bei der Aufnahme der Bild-Zeit-Daten anzupassen, indem beispielsweise ein Shutter, welcher ein Teil des Mittels zur Bilderfassung ist, länger oder kürzer geöffnet und so die Belichtungszeit angepasst wird. Vorzugsweise handelt es sich beim Shutter um einen elektronischen Shutter als Teil des Mittels zur digitalen Bilderfassung.

Weiterhin kann die Helligkeit in den Bild-Zeit-Daten rechnerisch angepasst werden. Auf diese Weise kann sichergestellt werden, dass sich alle Helligkeitswerte im Bild in einem definierten Bereich befinden, beispielsweise unter 200, unter der Annahme, dass 0 der kleinsten und 255 der grössten Helligkeit entspricht.

Eine weitere Möglichkeit besteht darin, den rechnerischen Helligkeitsabgleich für eine oder mehrere bestimmte Substanzen zu definieren. Dieses ist insbesondere für Substanzen geeignet, welche als Justier- und/oder Kalibriersubstanz verwendet werden. Auf diese Weise können gleichartige Kontrastverhältnisse für diese Substanzen geschaffen werden, also dass der Kontrast zwischen einer vorgegebenen Substanz und dem Hintergrund an diese Substanz angepasst ist und beispielsweise bei Wiederholungsmessungen im Wesentlichen gleich ist. Die Verknüpfung zwischen Helligkeit und einer bestimmten Substanz kann beispielsweise in einer Datenbank in der Steuereinheit abgelegt werden.

Im Laufe der Messung findet aufgrund der Erwärmung eine zeitliche Veränderung der Probe statt. Dieses kann insbesondere eine Tropfenbildung sein, welcher Tropfen sich in Abhängigkeit der zu analysierenden Substanz beim Erwärmen bildet und abreisst oder welcher sich durch die Erwärmung ausdehnt und insbesondere eine Längenänderung erfährt. Die Temperatur bei welcher der Tropfen abreisst bezeichnet den Tropfpunkt bzw. die Temperatur bei welcher der Tropfen eine vorgegebene Länge überschreitet den Erweichungspunkt. Für die meisten Substanzen kann entweder ein Tropfpunkt oder ein Erweichungspunkt bestimmt werden. Die zeitliche Veränderung der Probe wird anhand der Bild-Zeit-Daten ermittelt. Dazu kann über eine entsprechende Bildauswertung ermittelt werden, wann und bei welcher Temperatur, also zu welchem Zeitpunkt ein Tropf-Ereignis stattgefunden hat oder wann und bei welcher Temperatur der sich bildende Tropfen die vorgegebene Länge erreicht hat.

Damit diese Bestimmung mit einer möglichst guten und hohen Reproduzierbarkeit durchgeführt werden kann, kann das Verfahren weiterhin die Bestimmung eines Koordinaten-Ursprungs oder Koordinaten-Nullpunkts umfassen. Bei diesem Schritt wird mindestes ein vorgegebener Punkt des ermittelten Bildes als Koordinaten-Ursprung festgelegt und die Bestimmung der zeitlichen Veränderung der Probe in Relation zu diesem Koordinaten-Ursprung durchgeführt. Ein bevorzugter Fixpunkt zur Festlegung des Koordinaten-Ursprungs ist beispielsweise die Position der Probenkammer und insbesondere eines Ofens, welcher an die Probenkammer angrenzt und diese zumindest teilweise umschliesst. Diese oder eine andere im Messgerät fixierte Position kann in den Bild-Zeit-Daten der leeren Probenkammer vorzugsweise automatisch erkannt werden und als Koordinaten-Ursprung festgelegt werden.

Die Bild-Zeit-Daten und/oder die Temperatur-Zeit-Daten können kontinuierlich, in vorbestimmten Zeitintervallen und/oder zu vorgegebenen Zeitpunkten erfasst werden.

Anhand der zeitlichen Veränderung der Probe in Abhängigkeit der Temperatur kann beispielsweise der Tropfpunkt, der Erweichungspunkt und/oder die Viskosität als Stoffeigenschaft und/oder rheologische Eigenschaft ermittelt werden.

Die Ist-Temperatur, ein Auswertebereich, der Messzeitpunkt und/oder eine Längenskala, welche sich insbesondere auf den festgelegten Koordinaten-Ursprung bezieht, können in den Bild-Zeit-Daten angezeigt und auf diese Weise mit den Bild-Zeit-Daten verknüpft werden. Dazu können beispielsweise eine Temperaturanzeige, der Auswertebereich und/oder eine Skala physisch so im Messgerät angeordnet sein, dass diese bei der Aufnahme der Bild-Zeit-Daten erfasst werden. Vorzugsweise können die Ist-Temperatur, der Auswertebereich, der Messzeitpunkt und/oder die Längenskala in die Bild-Zeit-Daten eingeblendet werden und auf diese Weise miteinander visuell verbunden werden, so dass die Daten insbesondere beim Abspielen zuvor gespeicherter Daten miteinander verknüpft angezeigt werden. Die gemeinsame Anzeige von Bild-Zeit-Daten, Ist-Temperatur, Auswertebereich, Messzeitpunkt und/oder Längenskala ist sowohl benutzerfreundlich, als auch für die Nachvollziehbarkeit und Nachverfolgbarkeit der Messungen sehr vorteilhaft. Der Messzeitpunkt kann beispielsweise über eine dem Messgerät zugeordnete Uhr erfasst und in die Bild-Zeit-Daten eingeblendet werden. Weiterhin kann der Messzeitpunkt über die Sampling-Rate, also vorgegebene, zeitlich beabstandete Mess-Zeitpunkte, ermittelt werden.

Das Messgerät kann ferner eine Kühlvorrichtung umfassen oder mit einer Kühlvorrichtung zusammenwirken, so dass die Probe in der Probenkammer auch gekühlt werden kann. Diese Ausgestaltung ist vorteilhaft, wenn die Erstarrungstemperatur der zu analysierenden Substanz unterhalb der Raumtemperatur liegt und vor der Bestimmung des Tropf- oder Erweichungspunkts abgekühlt werden muss. Vorzugsweise werden derartige Substanzen in vorgekühlte Probengefässe gefüllt und bis zum Beginn der Messung oder zumindest bis zum Einsetzen in die Probenkammer auf Temperaturen unterhalb ihrer Erstarrungstemperatur gekühlt. Die Kühlvorrichtung kann als separates Bauteil ausgebildet sein oder eine gemeinsame Einheit mit der Heizvorrichtung bilden.

Als Probengefässe werden vorzugsweise kleine Metall-Tiegel mit einer standardisierten Form verwendet, wie sie beispielsweise in den bereits angeführten Normen beschrieben werden. Die Probengefässe für die Tropfpunkt- und die Erweichungspunkt-Bestimmung weisen einen Einlass und einen Auslass auf und unterscheiden sich vor allem im Durchmesser des Auslasses, durch welchen die Probe beim Erwärmen als Tropfen austritt.

Das Mittel zur Bilderfassung ist als Mittel zur digitalen Bilderfassung ausgestaltet und kann insbesondere eine CMOS-Kamera, eine CCD-Kamera (charge-couple-device), eine andere bekannte digitale Videokamera oder ein anderes geeignetes Halbleiterelement zur Bilderfassung sein.

Nach Einstellen eines gewünschten Messablaufs oder Messprogramms, insbesondere der Starttemperatur und der gewünschten Temperatur-Zeit-Sollwerte, kann detektiert werden, dass die Probe in die Probenkammer eingebracht wurde, und dadurch die Messung der Temperatur-Zeit-Daten und/oder die Erfassung der Bild-Zeit-Daten durch Einbringen der Probe in die Probenkammer automatisch gestartet werden. Vorteilhafterweise werden ein oder mehrere gefüllte Probengefässe in einem Probenhalter angeordnet, welcher ein Erkennungsmerkmal umfasst. Das Erkennungsmerkmal ist insbesondere so ausgestaltet, dass dieses über die Bild-Zeit-Daten und/oder die Steuereinheit erkannt wird und so die Messung auslöst oder startet.

Die Auswertung der gemessenen Bild-Zeit-Daten und Temperatur-Zeit-Daten kann online geschehen, wobei die Daten auch nach Abschluss der Messung, insbesondere offline, ausgewertet werden können.

Die Steuereinheit kann mindestens einen Lese-Schreib-Speicher umfassen, in welchem sowohl die vorgegeben Temperatur-Zeit-Sollwerte, als auch die ermittelten Temperatur-Zeit-Istwerte sowie die Bild-Zeit-Daten gespeichert werden können. Auf diese Weise können die Messergebnisse dokumentiert werden und zudem für eine oder mehrere weitere Auswertungen herangezogen werden.

Vorzugsweise ist zudem in der Steuereinheit ein Programm zur Durchführung des Verfahrens hinterlegt.

Das erfindungsgemässe Verfahren kann gleichzeitig an mindestens zwei in der Probenkammer befindlichen Substanzen durchgeführt werden, wodurch die Messunsicherheit verringert werden kann. Dazu kann das Messgerät einen Probenhalter umfassen, welcher zwei oder mehr Proben aufnehmen kann. Der Probenhalter kann zusammen mit den Proben in das Messgerät eingesetzt werden.

Ein Messgerät zu Durchführung des erfindungsgemässen Verfahrens wird im Anspruch 13 definiert und umfasst eine Probenkammer, einen Temperaturfühler, welcher die Temperatur in der Probenkammer erfasst, eine Heizvorrichtung, ein Mittel zur Bereitstellung von Temperatur-Zeit-Sollwerten, wobei die Heizvorrichtung die Probenkammer gemäss den vorgegebenen Temperatur-Zeit-Sollwerten aufheizt, ein Mittel zur Bilderfassung, welche das Innere der Probenkammer visuell erfasst und eine Steuereinheit mit einer Prozessoreinheit, wobei eine in der Probenkammer bereitgestellte Probe mit der Heizvorrichtung gemäss den vorgegebenen Temperatur-Zeit-Sollwerten aufgeheizt werden kann. Mit dem Temperaturfühler können Temperatur-Zeit-Istwerte und mit dem Mittel zur Bilderfassung Bild-Zeit-Daten erfasst und die zeitliche Veränderung der Probe in Abhängigkeit der Temperatur ermittelt werden. Die zeitliche Veränderung der Probe umfasst eine Bildung mindestens eines Tropfens sowie dessen Längenänderung oder Abreissen aufgrund einer Erweichung der Probe durch die Wärmezufuhr der Heizvorrichtung (11), welche in den Bild-Zeit-Daten wiedergegeben wird. Die zeitliche Veränderung der Probe ermöglicht die Bestimmung des Tropf- oder Erweichungspunkts der Probe.

Zur Durchführung des Verfahrens an zwei oder mehr Proben kann das Messgerät zudem einen Probenhalter mit mindestens zwei Aufnahmen umfassen. Weiterhin kann der Probenhalter für jede Probe ein Haltemittel umfassen.

Vorzugsweise weist der Probenhalter zudem ein Erkennungsmittel auf, welches eine automatische Erkennung der Art des Probenhalters, beispielsweise der Anzahl der einsetzbaren Proben und/oder ob es sich um einen Probenhalter für die Tropfpunkt- oder Erweichungspunktbestimmung handelt. Das Erkennungsmittel kann nach dem Einsetzen über das Mittel zur Bilderfassung erfasst werden und kann dazu verwendet werden, eine vorgegebene Messung automatisch zu starten.

Das erfindungsgemässe Verfahren sowie ein zur Durchführung geeignetes Messgerät werden anhand der folgenden Figuren näher beschrieben, wobei gleiche Elemente mit denselben Bezugszeichen versehen sind. Es zeigen:
- Fig. 1: Eine Seitenansicht eines Messgerät mit einer Probenkammer, wobei das Messgerät teilweise im Schnitt dargestellt ist;
- Fig. 2: eine schematische Darstellung der Probenkammer;
- Fig. 3A: einen Schnitt durch die Probenkammer ohne Probenhalter entlang der Line A-A in Figur 1;
- Fig. 3B: einen Schnitt durch die Probenkammer ohne Probenhalter entlang der Line B-B in Figur 1;
- Fig. 4: eine schematische, dreidimensionale Darstellung eines Probenhalters;
- Fig. 5: eine schematische Darstellung einer Aufnahme vom Inneren der Probenkammer während einer Bestimmung des Erweichungspunkts;
- Fig. 6: eine schematische Darstellung einer Aufnahme vom Inneren der Probenkammer während einer Bestimmung des Tropfpunkts.

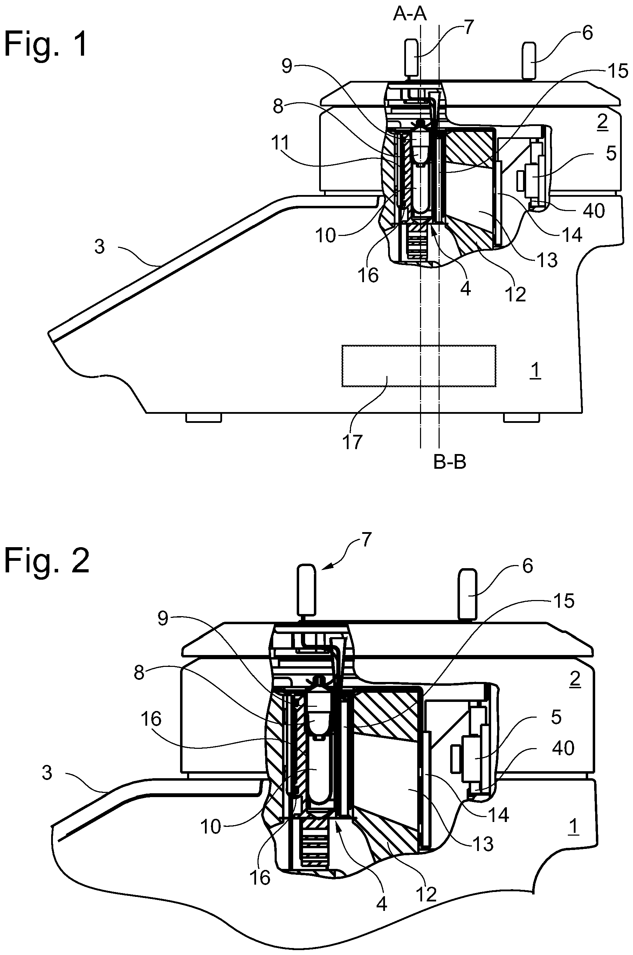

Figur 1 zeigt ein Messgerät zur Durchführung des erfindungsgemässen Verfahrens und Figur 2 eine Vergrösserung einer Probenkammer im Messgerät. Die Figuren 1 und 2 werden im Folgenden gemeinsam beschrieben. Das Messgerät umfasst ein Gehäuse 1 und einen Gehäuseteil 2. Im Gehäuse 1 sind unter anderem eine Steuereinheit 17 und weitere elektronische Komponenten angeordnet, welche hier nicht explizit gezeigt sind. Das Gehäuse 1 weist zudem auf einer Seite eine abgeschrägte Fläche mit einer Bedien- und/oder Anzeigeinheit 3 auf. Das Gehäuseteil 2 ist mit dem Gehäuse 1 verbunden. Das Gehäuseteil 2 ist mit einem Verschluss 6 ausgestattet, welcher eine Platte und einen Griff umfasst und horizontal verschoben werden kann, wodurch ein Zugang zur Probenkammer 4 geöffnet oder verschlossen werden kann. Weiterhin sind im Gehäuseteil 2 eine Probenkammer 4, ein Mittel zur digitalen Bilderfassung 5 und eine geeignete Lichtquelle 40, insbesondere einer LED-Lampe, angeordnet. Zur Durchführung einer Messung kann, wie hier gezeigt ein Probenhalter 7 in der Probenkammer angeordnet werden. Im Probenhalter 7 befinden sich ein Probengefäss 8 auf dem ein Deckel 9 liegt und ein Auffangbehälter 10. Das Probengefäss 8 weist einen Einlass und einen Auslass auf, durch welchen Auslass die Probe beim Erwärmen als Tropfen austritt. Der Probenhalter 7 kann auch zur Aufnahme mehrerer Proben ausgestaltet sein.

An mindestens einer Seite der Probenkammer 4 ist eine Heizvorrichtung 11, vorzugsweise eine Flachheizung, angeordnet, mit welcher die Probenkammer 4 und somit eine Probe anhand vorgegebener Temperatur-Zeit-Sollwerte erwärmt werden kann. Zur Messung von Temperatur-Zeit-Istwerten ist in der Probenkammer 4 zudem ein Temperaturfühler 16 angeordnet.

Die zum digitalen Bilderfassungsmittel 5 ausgerichtete Fläche der Probenkammer 4 weist ein Fenster 15 auf, durch welches das Mittel zur digitalen Bilderfassung 5 Bild-Zeit-Daten vom Inneren der Probenkammer 4 aufnehmen kann. Zwischen dem Mittel zur digitalen Bilderfassung 5 und dem Fenster 15 ist eine Isolation 12 der Probenkammer 4 angeordnet, in welcher ein Kanal oder eine Aussparung 13 ausgebildet ist. Das Mittel zur digitalen Bilderfassung 5 wird gegen den Kanal 13 von einem weiteren Fenster 14 geschützt.

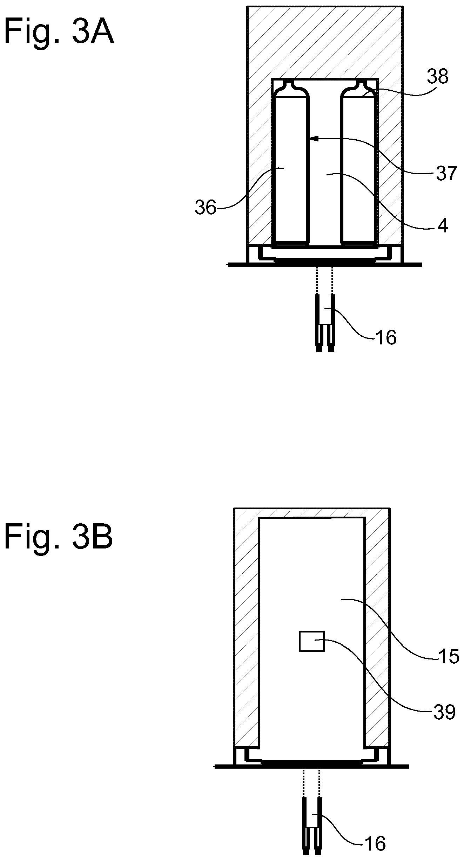

Figuren 3A und 3B zeigen beide einen Schnitt durch die Probenkammer 4 ohne Probenhalter entlang der Linie A-A bzw. B-B, welche in Figur 1 gezeigt sind.

In Figur 3A sind zwei Aussparungen 36 für zwei Probengefässe mit Deckel und Auffangbehälter zu erkennen. Weiterhin ist der Anschluss des Temperaturfühlers 16 zu sehen, welcher in dieser Figur hinter der Bildebene angeordnet ist. Charakteristische Merkmale der leeren Probenkammer 4 werden im erfindungsgemässen Verfahren zur Bestimmung des Koordinaten-Ursprungs herangezogen. Für die Bestimmung der horizontalen Positionierung können beispielsweise eine oder beide Innenkanten 37 der Aussparungen 36 herangezogen werden, welche in einem von der leeren Probenkammer 4 erfassten Bild als relativ scharf abgegrenzte senkrechte Schatten zu erkennen sind. Für die vertikale Positionierung wird mindestens einer der oberen Anschläge 38 verwendet, an welche die Probengefässe mit Deckel und Auffangbehälter anschlagen, wenn sie eingesetzt sind. Die Anschläge 38 bilden sich in den Bilddaten als scharfe horizontale Linien ab. Die Position des Schattens mindestens einer der Innenkanten 37 wird als horizontale Komponente des Koordinaten-Ursprungs und der des Anschlags 36 als vertikale Komponente erfasst und die Bilderkennung bzw. das erfindungsgemässe Verfahren darauf ausgerichtet.

Figur 3B zeigt einen Schnitt durch die Probenkammer entlang der Line B-B, welche in Bezug auf das Mittel zur digitalen Bilderfassung (5 in Figur 1) vor dem Fenster 15 liegt. Auf dem Fenster 15 ist eine hier rechteckig ausgestaltete Farbfläche 39 einer vorgegebenen Farbe, hier weiss, zu erkennen, welche im erfindungsgemässen Verfahren zur Durchführung des Weiss- und/oder Farbabgleichs erfasst und als bestimmte Farbe registriert wird. Anschliessend werden die Farben der weiteren Bilder digital oder rechnerisch an den vorgegebenen Wert der Farbfläche, beispielsweise RGB 100 100 100 angepasst. Das Fenster 15 ist durchsichtig und wurde hier in Figur 3B zur Vereinfachung undurchsichtig dargestellt.

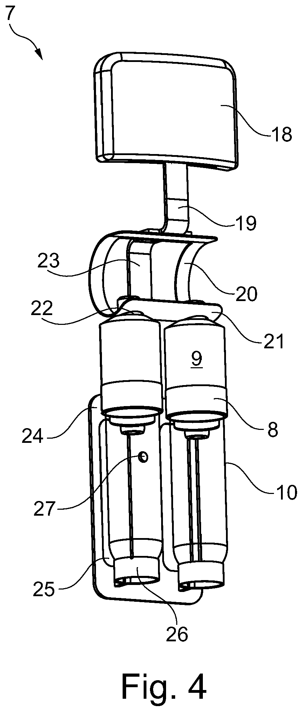

Figur 4 zeigt eine schematische, dreidimensionale Darstellung eines Probenhalters 7 für zwei Proben. Die zu analysierende Substanz kann in ein Probengefäss 8 gefüllt werden, welches auf einen transparenten Auffangbehälter 10 aufgesetzt wird. Das Probengefäss 8 weist einen Einlass und einen Auslass auf, durch welchen Auslass die Probe beim Erwärmen als Tropfen austritt. Auf dem Probengefäss 8 wird zudem ein Deckel 9 angeordnet. Der Auffangbehälter 10, das Probengefäss 8 und der Deckel 9 sind aufeinandergesetzt, jedoch hier nicht fest miteinander verbunden.

Der Probenhalter 7 umfasst einen Griff 18, welcher über einen ersten Steg 19 mit einer gebogenen Klammer, welche aus zwei Federelementen 20 besteht, verbunden ist. Die freien Enden der Federelemente 20 sind über einen Quersteg 21 miteinander verbunden, welcher zwei Aussparungen 22 für einen Teil des Deckels 9 umfasst. Weiterhin ist am ersten Steg 19 ein weiterer Steg 23 angeordnet, welcher sich vom Griff 18 senkrecht weg erstreckt und welcher in einem spatenartigen Element 24 endet. Das spatenartige Element 24 hat zwei senkrechte Aussparungen 25 und zwei ringförmige Halterungen 26, in welche die Auffangbehälter 10 eingesetzt werden können. Die senkrechten Aussparungen 25 definieren den vom Mittel zur digitalen Bilderfassung erfassten Messbereich. Der im Messgerät eingesetzte Probenhalter 7 ist so orientiert, dass die Probengefässe 8 vom Mittel zur digitalen Bilderfassung weg gerichtete sind. Die Auffangbehälter 10 sind wie hier gezeigt transparent, so dass das zeitliche Verhalten der Probe, wie eine Tropfenbildung aufgrund der Erwärmung der Probe, mit dem Mittel zur digitalen Bilderfassung erfasst werden kann.

Ein Erkennungsmittel 27 kann in der Mittelstrebe des spatenförmigen Elements 24 angeordnet sein. Das Erkennungsmittel 27 ist hier als Loch ausgestaltet und dient der automatischen Erkennung des Probenhalters beim Einsetzen in die Probenkammer durch das Mittel zur digitalen Bilderfassung. Das Erkennungsmittel 27 kann verschiedene Grössen, Formen und/oder Positionen auf dem Probenhalter 7 haben, so dass bei der Erkennung zwischen verschiedenen Probenhaltern 7 unterschieden werden kann. Selbstverständlich kann der Probenhalter auch mehrere gleichartige oder unterschiedliche Erkennungsmittel umfassen. Als Erkennungsmittel können neben dem hier gezeigten Loch auch mehrere Löcher, andere markante Kennzeichen sowie maschinenlesbare Codes, wie Barcodes oder Smartcodes, oder elektronisch erfassbare Kennzeichen, wie beispielsweise RFID-Tags eingesetzt werden.

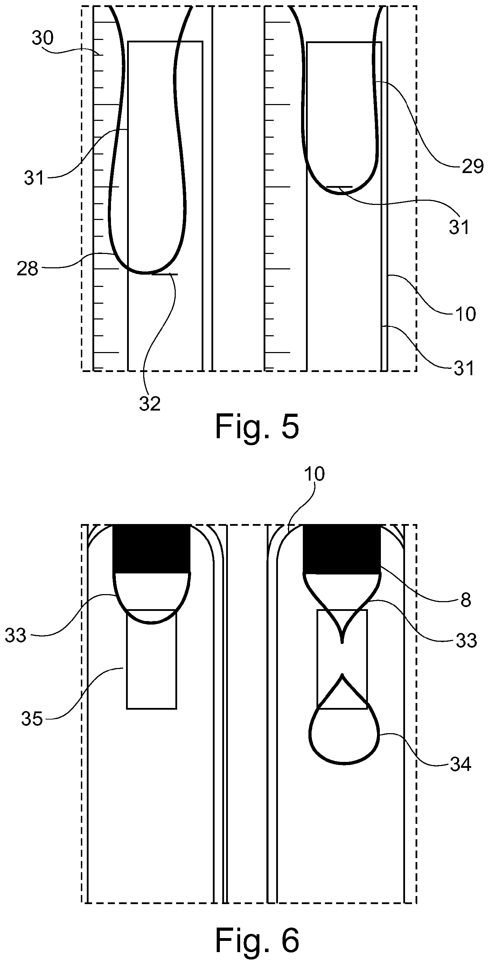

Figuren 5 und 6 zeigen schematisch Aufnahmen vom Inneren der Probenkammer während einer Bestimmung des Erweichungs- oder des Tropfpunktes.

Zur Bestimmung des Tropfpunktes oder des Erweichungspunktes, wird in einem Ausführungsbeispiel nach dem Einschalten des Messgeräts ein Weiss- oder Farbabgleich durchgeführt, um das Farbspektrum des Mittels zur digitalen Bilderfassung anzupassen und/oder einzustellen. Dazu ist in der Probenkammer ein Bereich mit einer definierten Farbe, vorzugsweise weiss, angeordnet (s. Figur 3B). Dieser Bereich wird vor dem Einbringen der Proben erfasst und das Mittel zur digitalen Bilderfassung derart eingestellt, dass das erfasste Bild dieses Bereiches einer vorgegebenen Farbe entspricht, beispielsweise R 100 G 100 B 100, des RGB Farbschemas. Der Farb- und/oder Weissabgleich ermöglicht die Erfassung von vergleichbaren Bild-Zeit-Daten, welche insbesondere aufgrund der gezeigten Farben vergleichbar sind. Zudem ermöglicht der Weiss- und/oder Farbabgleich Änderungen der zur Beleuchtung der Probenkammer verwendeten Lichtquelle, hier einer LED-Lampe, zu kompensieren.

Anschliessend wird ein Koordinaten-Ursprung oder Koordinaten-Nullpunkt ermittelt, damit die Bestimmung mit einer möglichst guten und hohen Reproduzierbarkeit durchgeführt werden kann. Bei diesem Schritt wird mindestes ein vorgegebener Punkt des ermittelten Bildes als Koordinaten-Ursprung festgelegt und die Bestimmung der zeitlichen Veränderung der Probe in Relation zu diesem Koordinaten-Ursprung durchgeführt. Ein bevorzugter Fixpunkt zur Festlegung des Koordinaten-Ursprung ist beispielsweise die Position der Probenkammer. Der Koordinaten-Ursprung sollte anhand eines charakteristischen Merkmals im Bild ermittelt werden, welches leicht zu erkennen ist und sowohl eine horizontale als auch eine vertikale Positionsermittlung erlaubt, siehe Figur 3A.

Ein Helligkeitsabgleich kann vor oder nach der Ermittlung des Koordinaten-Ursprungs durchgeführt werden, wobei vorzugsweise zuerst der Koordinaten-Ursprung ermittelt wird. Der Helligkeitsabgleich dient dazu sicherzustellen, dass die Bild-Zeit-Daten bei im Wesentlichen gleichen Helligkeitsverhältnissen erfasst werden. Auf diese Weise kann die Reproduzierbarkeit von Messungen gleichartiger Proben zu verschiedenen Zeitpunkten verbessert werden. Der Helligkeitsabgleich kann auf eine der beschriebenen Methoden durchgeführt werden. Vorzugsweise werden die Belichtungszeiten angepasst.

Vorzugweise wird sowohl der Farbabgleich, der Helligkeitsabgleich als auch die Bestimmung des Koordinaten-Ursprungs automatisch nach dem Aufstarten des Messgeräts durchgeführt. Dieses kann in der hier dargestellten oder in einer beliebigen Reihenfolge geschehen. Dazu ist in der Steuereinheit, genauer der darin befindlichen Prozessoreinheit, ein entsprechendes Programm hinterlegt.

Nun ist das Messgerät messbereit und es kann mindestens eine Probe vorbereitet und vorzugsweise in ein für die jeweilige Messung geeignetes Probengefäss eingefüllt werden. Das Probengefäss kann anschliessend in einen Probenhalter eingesetzt werden, wie er im Zusammenhang mit Figur 4 beschrieben wurde. Häufig werden zwei oder mehr Proben gleichzeitig vermessen.

Vor dem Einsetzen des Probenhalters in das Messgerät, können vom Benutzer die Mess- und Probenparameter bestimmt und über die Anzeige- und/oder Bedieneinheit eingegeben werden. Diese können beispielsweise Informationen zur Substanz, die vorzugebenden Temperatur-Zeit-Sollwerte, die Starttemperatur und weitere für den Benutzer relevante Informationen umfassen.

Nachdem der Probenhalter mit der mindestens einen Probe in das Messgerät eingesetzt wurde, kann nochmals ein probenspezifischer Helligkeitsabgleich durchgeführt werden, so dass gleichartige Kontrastverhältnisse für die zu analysierende Substanz geschaffen werden, also dass der Kontrast zwischen Substanz und Hintergrund im Wesentlichen gleich ist. Die Verknüpfung zwischen Helligkeit und einer bestimmten Substanz kann beispielsweise in einer Datenbank in der Steuereinheit abgelegt werden. Dieses kann für Justier- und/oder Kalibriersubstanzen bereits werksseitig vorgegeben werden oder durch den Benutzer festgelegt werden, was sich insbesondere bei Reihenmessungen oder für Messungen im Bereich der Qualitätssicherung eignet, wenn häufig gleiche oder sehr ähnliche Substanzen bzw. Proben vermessen werden.

Die Messung kann durch den Benutzer ausgelöst werden oder automatisch durch Erkennung eines Erkennungsmittels am Probenhalter gestartet werden. Die Messung umfasst die Auswahl der Messmethode, also der Tropfpunkt- oder Erweichungspunktbestimmung, das Einstellen der gewünschten Starttemperatur und Aufheizen der Probenkammer auf diese Temperatur. Anschliessend heizt die Heizvorrichtung die Probenkammer und damit die Probe gemäss vorgegebenen Temperatur-Zeit-Sollwerten auf. Gleichzeitig werden zu vorbestimmten Zeitpunkten oder kontinuierlich die Temperatur-Zeit-Daten sowie die Bild-Zeit-Daten erfasst und vorzugsweise abgespeichert.

Aus diesen Daten kann nun die zeitliche Veränderung der Probe ermittelt werden, dieses geschieht nahezu in Echtzeit und/oder kann nachträglich anhand der aufgenommenen und abgespeicherten Daten ermittelt werden.

Aufgrund der eingestellten Parameter und der ausgewählten Messmethode wird aus der zeitlichen Veränderung der Probe das erste Tropfevent ermittelt und die zugehörige Temperatur als Tropfpunkt erfasst oder es wird die Temperatur als Erweichungspunkt erfasst, an welcher der erste Tropfen eine vorgegebene Länge erreicht.

In einem weiteren Schritt können zudem eine oder mehrere rheologische Eigenschaften der Probe aus deren zeitlicher Veränderung ermittelt werden. Bereits die Analyse der Bild-Zeit-Daten kann dem Benutzer Aufschluss über das temperaturabhängige rheologische Verhalten der zu analysierenden Substanz geben.

In Figur 5 sieht man das Schema einer typischen Aufnahme für die Bestimmung des Erweichungspunkts. Die Aufnahme zeigt das Innere der Probenkammer, genauer den Bereich in dem die Auffangbehälter angeordnet sind. In der Probenkammer sind zwei Proben 28, 29 angeordnet, welche sich aufgrund des Erwärmens ausgedehnt und jeweils einen Tropfen ausgebildet haben. Über der Aufnahme des Tropfens ist eine Skala 30 eingeblendet und ein Auswertebereich 31 durch einen eingeblendeten Rahmen angezeigt. Aus den erfassten Bild-Zeit-Daten wird die Länge des Tropfens bestimmt, was dem Anwender zudem durch Anzeige eines horizontalen Strichs 32 angezeigt wird, welcher immer das untere Ende des Tropfens markiert. Sobald der Tropfen eine vorgegebene Länge erreicht hat, wird die zugehörige Temperatur als Erweichungspunkt registriert.

Figur 6 zeigt ebenfalls schematisch eine Aufnahme aus dem Inneren der Probenkammer bei einer Tropfpunktbestimmung. Es ist wiederum der vom Mittel zur Bildbearbeitung erfasste Bereich, welcher die Auffangbehälter umfasst, gezeigt. Die Aufnahme stellt den Zeitpunkt des Erreichens des Tropfpunkts dar. Dieser wird erfasst, in dem das angezeigte Auswertungsfenster 35 digital erfasst und das Abreissen eines Tropfens 34 von der Substanz 33 registriert wird. Die zugehörige Temperatur wird dann als Tropfpunkt erfasst.

Zusätzlich kann aus den ermittelten Temperatur-Zeit-Istwerten und den Bild-Zeit-Daten auch eine rheologische Eigenschaft der Substanz ermittelt werden, insbesondere die Viskosität. Dazu kann beispielsweise die Tropffrequenz bestimmt und ausgewertet werden.

**Bezugszeichenliste**

| | | | |
|---|---|---|---|
| 1 | Gehäuse | 22 | Aussparung |
| 2 | Gehäuseteil | 23 | Steg |
| 3 | Bedien- und/oder Anzeigeeinheit | 24 | spatenförmiges Element |
| 4 | Probekammer | 25 | Aussparung |
| 5 | Mittel zur digitalen Bilderfassung | 26 | Halterung |
| 6 | Verschluss | 27 | Erkennungsmittel |
| 7 | Probenhalter | 28 | Tropfen |
| 8 | Probengefäss | 29 | Tropfen |
| 9 | Deckel | 30 | Skala |
| 10 | Auffangbehälter | 31 | Auswertungsrahmen |
| 11 | Heizvorrichtung | 32 | Strich |
| 12 | Isolierung | 33 | Substanz |
| 13 | Kanal / Aussparung | 34 | Tropfen |
| 14 | Fenster | 35 | Auswertebereich |
| 15 | Fenster | 36 | Aussparung |
| 16 | Temperaturfühler | 37 | Innenkante |
| 17 | Steuereinheit | 38 | Anschlag |
| 18 | Griff | 39 | Farbfläche oder Farbbereich |
| 19 | Steg | 40 | Lichtquelle |
| 20 | Federelement | | |
| 21 | Quersteg | | |

## Patentansprüche

1. Verfahren zur Bestimmung des Erweichungs- oder Tropfpunkts einer Substanz mit einem Messgerät, welches eine Probenkammer (4), einen Temperaturfühler (16), eine Heizvorrichtung (11), ein Mittel zur Bereitstellung von Temperatur-Zeit-Sollwerten, ein Mittel zur Bilderfassung (5) und eine Steuereinheit (17) mit einer Prozessoreinheit umfasst; wobei die Heizvorrichtung (11) die Probenkammer (4) aufheizt; wobei der Temperaturfühler (16) die Temperatur in der Probenkammer (4) erfasst; und wobei das Mittel zur Bilderfassung (5) das Innere der Probenkammer (5) visuell erfasst; wobei das Verfahren die folgenden Schritte umfasst:
a. Bereitstellen eines Probengefässes (8) mit einer Probe der zu analysierenden Substanz und Einbringen des Probengefässes (8) in die Probenkammer (4);
b. Einstellen der Probenkammer (4) auf eine vorgegebene Starttemperatur;
c. Bereitstellen von Temperatur-Zeit-Sollwerten und Aufheizen der Probenkammer (4) mit der Heizvorrichtung (11) gemäss den vorgegebenen Temperatur-Zeit-Sollwerten;
d. Messen von Temperatur-Zeit-Istwerten in der Probenkammer (4) mit dem Temperaturfühler (16) und Erfassen von Bild-Zeit-Daten aus dem Inneren der Probenkammer (4) mit dem Mittel zur Bilderfassung (5);
e. Ermitteln der zeitlichen Veränderung der Probe in Abhängigkeit der Temperatur anhand der erfassten Bild-Zeit-Daten und Temperatur-Zeit-Istwerte, welche die Bildung mindestens eines Tropfens sowie dessen Längenänderung oder Abreissen aufgrund einer Erweichung der Probe durch die Wärmezufuhr der Heizvorrichtung (11) umfasst; und
f. Ermitteln des Tropfpunkts oder des Erweichungspunkts der Probe anhand ihrer zeitlichen Veränderung in Abhängigkeit der Temperatur;
und wobei das Verfahren ferner einen Farb- und/oder Weissabgleich umfasst, wobei das vom Mittel zur Bilderfassung (5) wiedergegebene Farbspektrum anhand einer in der Probenkammer (4) angeordneten Fläche (39) mit einer definierten Farbe angepasst wird.

2. Verfahren nach Anspruch 1, **dadurch gekennzeichnet, dass** die Bild-Zeit-Daten der Substanz während des Erwärmungsprozesses zur Erkennung von Messfehlern aufgrund von sich atypisch verhaltenden Proben optisch kontrolliert werden.

3. Verfahren nach Anspruch 1 oder 2, **dadurch gekennzeichnet, dass** dieses einen Helligkeitsabgleich für die Erfassung der Bild-Zeit-Daten umfasst.

4. Verfahren nach Anspruch 3, **dadurch gekennzeichnet, dass** der Helligkeitsabgleich durch Anpassung der Helligkeit einer regelbaren Lichtquelle (40) oder durch Anpassung einer Belichtungsdauer des Mittels zur Bilderfassung (5) erfolgt, oder dass der Helligkeitsabgleich ein rechnerischer Abgleich an den aufgenommenen digitalen Bild-Zeit-Daten ist.

5. Verfahren nach einem der Ansprüche 1 bis 4, **dadurch gekennzeichnet, dass** der Helligkeitsabgleich zudem für eine bestimmte Substanz ein Kontrastabgleich zwischen einem für die Substanz vorgegeben Wert und dem tatsächlichen Wert ist.

6. Verfahren nach einem der Ansprüche 1 bis 5, **dadurch gekennzeichnet, dass** dieses die Bestimmung eines Koordinaten-Ursprungs innerhalb der Probenkammer (4) umfasst.

7. Verfahren nach einem der Ansprüche 1 bis 6, **dadurch gekennzeichnet, dass** die Bild-Zeit-Daten eine Temperaturanzeige, einen Auswertebereich (35) und/oder eine Längenskala (30) enthalten, so dass die erfassten Bild-Zeiten-Daten zudem Informationen über die Isttemperatur in der Probenkammer, die Längenänderung und/oder das Tropfverhalten der Substanz enthalten.

8. Verfahren nach einem der Ansprüche 1 bis 7, **dadurch gekennzeichnet, dass** die Probenkammer (4) gekühlt und/oder geheizt wird, wobei das Messgerät zudem eine Kühlvorrichtung umfasst.

9. Verfahren nach einem der Ansprüche 1 bis 8, **dadurch gekennzeichnet, dass** die Bild-Zeit-Daten und/oder die Temperatur-Zeit-Daten in vorbestimmten Zeitintervallen oder kontinuierlich erfasst werden.

10. Verfahren nach einem der Ansprüche 1 bis 9, **dadurch gekennzeichnet, dass** die Schritte der Messung der Temperatur-Zeit-Daten und die Erfassung der Bild-Zeit-Daten durch Einbringen der Probe in die Probenkammer (4) automatisch gestartet werden.

11. Verfahren nach einem der Ansprüche 1 bis 10, **dadurch gekennzeichnet, dass** in der Prozessoreinheit der Steuereinheit (17) ein Programm hinterlegt ist, mit welchem das Überschreiten zumindest einer vorgegebenen Länge der Probe oder eines Tropfvorgangs automatisch anhand der Bild-Zeit-Daten erkannt und dem Benutzer die zugehörige Isttemperatur angezeigt wird.

12. Verfahren nach einem der Ansprüche 1 bis 11, **dadurch gekennzeichnet, dass** die erfassten Bild-Zeit-Daten und die Temperatur-Zeit-Daten in einem Schreib-LeseSpeicher der Prozessoreinheit gespeichert werden.

13. Messgerät zur Durchführung des Verfahrens gemäss einem der Ansprüche 1 bis 12 umfassend eine Probenkammer (4); ein Mittel zur Bereitstellung von Temperatur-Zeit-Sollwerten; eine Heizvorrichtung (11), welche die Probenkammer (4) gemäss den vorgegebenen Temperatur-Zeit-Sollwerten aufheizt; einen Temperaturfühler (16), welcher Temperatur-Zeit-Istwerte in der Probenkammer (4) erfasst; ein Mittel zur Bilderfassung (5), welches das Innere der Probenkammer (4) visuell erfasst und Bild-Zeit-Daten aufnimmt; und eine Steuereinheit (17) mit einer Prozessoreinheit, in welcher ein Programm zur Durchführung des Verfahrens gemäss einem der Ansprüche 1 bis 12 hinterlegt ist; wobei anhand der Temperatur-Zeit-Istwerte und der Bild-Zeit-Daten eine zeitliche Veränderung einer in der Probenkammer (4) angeordneten Probe ermittelt wird, wobei die zeitliche Veränderung der Probe die Bildung mindestens eines Tropfens sowie dessen Längenänderung oder Abreissen aufgrund einer Erweichung der Probe durch die Wärmezufuhr der Heizvorrichtung (11) umfasst, welche in den Bild-Zeit-Daten erfasst wird; und aus der zeitlichen Veränderung der Probe deren Tropf- oder Erweichungspunkt bestimmt wird.

14. Messgerät nach Anspruch 13, **dadurch gekennzeichnet, dass** dieses ferner einen Probenhalter (7) mit mindestens zwei Aufnahmen (22) zur Aufnahme von Proben, mindestens zwei Haltemitteln (26) für die Proben und mindestens ein Erkennungsmittel (27) umfasst.

## Claims

1. Method of determining the softening point or the dropping point of a substance with a measuring instrument that comprises a sample chamber (4), a temperature sensor (16), a heater device (11), a means for providing temperature/time target values, an image-recording means (5) and a controller unit (17) with a processor unit; wherein the heater device (11) heats the sample chamber (4), the temperature sensor (16) measures the temperature in the sample chamber (4), and the image-recording means (5) captures a visual image of the interior of the sample chamber (4); said method comprising the steps of:
a. providing a sample receptacle (8) with a sample of the substance that is to be analyzed and setting the sample receptacle (8) into the sample chamber (4);
b. setting the sample chamber (4) to a given starting temperature;
c. providing temperature/time target values and heating the sample chamber (4) with the heater device (11) in accordance with the given temperature/time target values;
d. measuring actual temperature/time values in the sample chamber (4) with the temperature sensor (16) and capturing image/time date from the interior of the sample chamber (4) with the image-recording means (5);
e. determining the change of the sample over time as a function of the temperature based on the recorded image/time data and actual temperature/time data, wherein said change of the sample comprises the formation of at least one drop as well as the change in length or breaking-away of the drop due to a softening of the sample as a result of the heat introduced by the heater device (11); and
f. determining the dropping point or the softening point of the sample based on the change over time of the sample as a function of the temperature;
and wherein the method further comprises a color-balance adjustment and/or a white-balance adjustment, wherein the color spectrum rendered by the image-recording means (5) is adjusted by means of a color reference area (39) of a defined color which is arranged in the sample chamber (4).

2. Method according to claim 1, **characterized in that** the image/time data of the substance are optically monitored during the heating process in order to detect measurement errors based on atypical behaviors of samples.

3. Method according to claim 1 or 2, **characterized in that** the method comprises a brightness adjustment for the recording of the image/time data.

4. Method according to claim 3, **characterized in that** the brightness adjustment is made by adjusting the brightness of an adjustable light source (40) or by adjusting an exposure time of the image-recording means (5), or through a mathematically calculated adjustment of the recorded digital image/time data.

5. Method according to one of the claims 1 to 4, **characterized in that** the brightness adjustment additionally comprises a substance-specific contrast adjustment between a given value for the substance and the actual value.

6. Method according to one of the claims 1 to 5, **characterized in that** the method comprises the determination of an origin of a coordinate system within the sample chamber (4).

7. Method according to one of the claims 1 to 6, **characterized in that** the image/time data comprise a temperature indication, an area of evaluation (35) and/or a length measurement scale (30), so that the recorded image/time data also include information regarding the actual temperature in the sample chamber, the change in length, and/or the drop-formation behavior of the substance.

8. Method according to one of the claims 1 to 7, **characterized in that** the sample chamber (4) is cooled and/or heated, wherein the measuring instrument additionally comprises a cooling device.

9. Method according to one of the claims 1 to 8, **characterized in that** the image/time data and/or the temperature/time data are recorded in predetermined time intervals or continuously.

10. Method according to one of the claims 1 to 9, **characterized in that** the steps of measuring the temperature/time data and recording the image/time data are started automatically by setting the sample into the sample chamber (4).

11. Method according to one of the claims 1 to 10, **characterized in that** a program is stored in the processor unit of the controller unit (17), said program being operable to automatically detect from the image/time data when at least a given length of the sample is exceeded or when a drop-formation event has occurred, and to indicate the associated actual temperature to the user.

12. Method according to one of the claims 1 to 11, **characterized in that** the recorded image/time data and the temperature/time data are stored in a read/write memory of the processor unit.

13. Measuring instrument, operable to perform the method according to one of the claims 1 to 12, comprising a sample chamber (4), a means for providing temperature/time target values, a heater device (11) to heat the sample chamber (4) in accordance with the predetermined temperature/time target values, a temperature sensor (16) which measures the actual temperature/time values in the sample chamber (4), an image-recording means (5) which captures a visual image of the interior of the sample chamber (4) and records image/time data, and a controller unit (17) with a processor unit in which a program is stored for the execution of the method according to one of the claims 1 to 12; wherein based on the actual temperature/time data and recorded image/time data a change over time is determined which occurs in a sample that is arranged in the sample chamber (4); wherein said change over time comprises the formation of at least one drop as well as the change in length or breaking away of the drop due to the heat introduced by the heater device (11) which is recorded in the image/time data; and wherein based on the change over time of the sample, the dropping- or softening point of the sample is determined.

14. Measuring instrument according to claim 13, **characterized in that** the measuring instrument further comprises a sample holder (7) with at least two seats (22) to hold samples, at least two holder means (26) for the samples and at least one means of identification (27).

## Revendications

1. Procédé de détermination du point de ramollissement ou de goutte d'une substance avec un appareil de mesure, lequel comprend une chambre d'échantillon (4), une sonde de température (16), un dispositif de chauffage (11), un moyen de mise à disposition de valeurs de consigne température-temps, un moyen de capture d'image (5), une unité de commande (17) avec une unité de processeur ; dans lequel le dispositif de chauffage (11) chauffe la chambre d'échantillon (4) ; dans lequel la sonde de température (16) saisit la température dans la chambre de sonde (4) ; et dans lequel le moyen de capture d'image (5) saisit visuellement l'intérieur de la chambre d'échantillon (5) ; dans lequel le procédé comprend les étapes suivantes :
a. mise à disposition d'un récipient d'échantillon (8) avec un échantillon de la substance à analyser et introduction du récipient d'échantillon (8) dans la chambre d'échantillon (4) ;
b. réglage de la chambre d'échantillon (4) sur une température de départ prédéfinie ;
c. mise à disposition de valeurs de consigne température-temps et chauffage de la chambre d'échantillon (4) avec le dispositif de chauffage (11) conformément aux valeurs de consigne température-temps prédéfinies ;
d. mesure de valeurs réelles température-temps dans la chambre d'échantillon (4) avec la sonde de température (16) et saisie de données image-temps de l'intérieur de la chambre d'échantillon (4) au moyen de la capture d'image (5) ;
e. détermination du changement temporel de l'échantillon en fonction de la température à l'aide des données image-temps saisies et des valeurs réelles température-temps, qui comprend la formation d'au moins une goutte ainsi que son changement de longueur ou détachement en raison d'un ramollissement de l'échantillon par l'apport de chaleur du dispositif de chauffage (11) ; et
f. détermination du point de goutte ou du point de ramollissement de l'échantillon à l'aide de son changement temporel en fonction de la température ;
et dans lequel le procédé comprend en outre une balance des couleurs et/ou blancs, dans lequel le spectre de couleurs rendu par le moyen de capture d'image (5) est adapté à l'aide d'une surface (39) agencée dans la chambre d'échantillon (4) avec une couleur définie.

2. Procédé selon la revendication 1, **caractérisé en ce que** les données image-temps de la substance sont contrôlées optiquement pendant le processus de chauffage pour la saisie d'erreurs de mesure en raison d'échantillons à comportement atypique.

3. Procédé selon la revendication 1 ou 2, **caractérisé en ce qu'**il comprend une comparaison de la luminosité pour la saisie des données image-temps.

4. Procédé selon la revendication 3, **caractérisé en ce que** la comparaison de la luminosité se fait par adaptation de la luminosité d'une source de lumière réglable (40) ou par adaptation d'une durée d'éclairage du moyen de capture d'image (5), ou que la comparaison de la luminosité est une comparaison arithmétique au niveau des données image-temps numériques enregistrées.

5. Procédé selon l'une quelconque des revendications 1 à 4, **caractérisé en ce que** la comparaison de la luminosité est en outre pour une substance déterminée une comparaison du contraste entre une valeur prédéfinie pour la substance et la valeur réelle.

6. Procédé selon l'une quelconque des revendications 1 à 5, **caractérisé en ce qu'**il comprend la détermination d'un point d'origine à l'intérieur de la chambre d'échantillon (4).

7. Procédé selon l'une quelconque des revendications 1 à 6, **caractérisé en ce que** les données image-temps contiennent un affichage de température, une zone d'évaluation (35) et/ou une échelle de longueur (30), de sorte que les données image-temps capturées contiennent en outre des informations sur la température réelle dans la chambre d'échantillon, le changement de longueur et/ou le comportement de goutte de la substance.

8. Procédé selon l'une quelconque des revendications 1 à 7, **caractérisé en ce que** la chambre d'échantillon (4) est refroidie et/ou chauffée, dans lequel l'appareil de mesure comprend en outre un dispositif de refroidissement.

9. Procédé selon l'une quelconque des revendications 1 à 8, **caractérisé en ce que** les données image-temps et/ou les données température-temps sont saisies dans des intervalles de temps prédéterminés ou en continu.

10. Procédé selon l'une quelconque des revendications 1 à 9, **caractérisé en ce que** les étapes de la mesure des données température-temps et la saisie des données image-temps démarre automatiquement par l'introduction de l'échantillon dans la chambre d'échantillon (4).

11. Procédé selon l'une quelconque des revendications 1 à 10, **caractérisé en ce qu'**un programme est déposé dans l'unité de processeur de l'unité de commande (17), avec lequel le dépassement d'au moins une longueur prédéfinie de l'échantillon ou d'un procédé de goutte est saisi automatiquement à l'aide des données image-temps et la température réelle associée est affichée à l'utilisateur.

12. Procédé selon l'une quelconque des revendications 1 à 11, **caractérisé en ce que** les données image-temps saisies et les données température-temps sont enregistrées dans une mémoire écriture-lecture de l'unité de processeur.

13. Appareil de mesure pour la réalisation du procédé selon l'une quelconque des revendications 1 à 12 comprenant une chambre d'échantillon (4) ; un moyen de mise à disposition de valeurs de consigne température-temps ; un dispositif de chauffage (11), lequel chauffe la chambre d'échantillon (4) conformément aux valeurs de consigne température-temps prédéfinies ; une sonde de température (16), laquelle saisit des valeurs réelles température-temps dans la chambre d'échantillon (4) ; un moyen de capture d'image (5), lequel saisit visuellement l'intérieur de la chambre d'échantillon (4) et enregistre des données image-temps ; et une unité de commande (17) avec une unité de processeur, dans laquelle un programme pour la réalisation du procédé selon l'une quelconque des revendications 1 à 12 est déposé ; dans lequel un changement temporel d'un échantillon agencé dans la chambre d'échantillon (4) est déterminé à l'aide des valeurs réelles température-temps et des données image-temps, dans lequel le changement temporel de l'échantillon comprend la formation d'au moins une goutte ainsi que son changement de longueur ou détachement en raison d'un ramollissement de l'échantillon par l'apport de chaleur du dispositif de chauffage (11), qui est saisi dans les données image-temps ; et le point de goutte ou de ramollissement de l'échantillon est déterminé à partir de son changement temporel.

14. Appareil de mesure selon la revendication 13, **caractérisé en ce qu'**il comprend en outre un porte-échantillon (7) avec au moins deux logements (22) pour la réception d'échantillons, au moins deux moyens de retenue (26) pour les échantillons et au moins un moyen d'identification (27).
